# EUROPÄISCHE PATENTSCHRIFT

(11) **EP 0 530 506 B1**
(45) Veröffentlichungstag und Bekanntmachung des Hinweises auf die Patenterteilung: **01.05.1996**
(21) Anmeldenummer: 92113099.3
(22) Anmeldetag: 31.07.1992
(51) Int. Cl.: C07D 263/18, C07F 9/02

(54) **Optisch aktive Omega-Halogen-2-amino-alkancarbonsäure-derivate, Verfahren zu ihrer Herstellung und ihre Verwendung zur Herstellung optisch aktiver phosphorhaltiger Alpha-Aminosäuren**
Optically active omega-halogen-2-amino alkanoic acids derivatives, process for their preparation and their use for the preparation of optically active phosphor-containing alpha-aminoacids
Dérivés optiquement actives des acides oméga-halogène-2-amino alkanoiques, procédé pour leur préparation et leur utilisation pour la préparation des phosphoro-alpha amino-acides optiquement actifs

(30) Priorität: 03.08.1991 DE 4125753
(43) Veröffentlichungstag der Anmeldung: 10.03.1993
(73) Patentinhaber: Hoechst Schering AgrEvo GmbH, D-13342 Berlin (DE)
(72) Erfinder: Zeiss, Hans-Joachim, Dr., W-6231 Sulzbach/Taunus (DE)

(56) Entgegenhaltungen:
- EP-A- 0 346 658
- TETRAHEDRON Bd. 44, Nr. 17, GREAT BRITAIN Seiten 5479 - 5486 DEREK H.R.BARTON ET AL. 'Manipulation of the carboxyl groups of alpha-Amino-acids and Peptides using radical chemistry based on esters of N-hydroxy-2-thiopyridone'
- J.CHEM.SOC.CHEM.COMMUN. 1989, LONDON Seiten 423 - 425 BARRIE W.BYCROFT ET AL. 'A Chiral synthesis of trans-Carbapenam-3-carboxylic Acid and the Assignment of (3S,5S) Configuration to the corresponding product from Serratia and Erwinia Species.'
- BULL. CHEM. SOC. JAPAN Bd. 60, Mai 1987, JAPAN Seiten 1761 - 1766 NOBUTO MINOWA ET AL. 'Asymmetric synthesis of (+)-Phosphinothricin and related compounds by the Michael Addition of Glycine Schiff bases to vinyl compounds.'
- SYNTHESIS 1989, STUTTGART Seiten 542 - 544 JOHN M.SCHOLTZ ET AL. 'A convenient differential protection strategy for functional group manipulation of Aspartic and Glutamic acids.'

## Beschreibung

Die Erfindung betrifft optisch aktive, cyclische (2S)- und (2R)-ω-Halogen-2-aminoalkancarbonsäure-derivate der Formel I sowie deren Gemische, 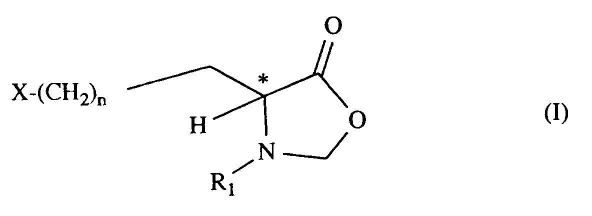
worin
- R₁: (C₁-C₆)Alkyl, das unsubstituiert oder durch ein oder mehrere Reste aus der Gruppe Hydroxy, (C₁-C₄)Alkoxy, [(C₁-C₄)Alkyl]-carbonyl und [(C₁-C₄)Alkoxy]-carbonyl substituiert ist, (C₂-C₆)Alkenyl, (C₂-C₆)Alkinyl oder eine Gruppe der Formel R₂-CO- oder R₃-SO₂-,
- R₂: (C₁-C₁₁)Alkyl, (C₆-C₁₃)Aryl oder substituiertes (C₆-C₁₃)Aryl, (C₁-C₆)Alkoxy, das unsubstituiert oder durch ein oder mehrere Reste, vorzugsweise einen Rest aus der Gruppe (C₆-C₁₃)Aryl und substituiertes (C₆-C₁₃)Aryl substituiert ist,
- R₃: (C₁-C₄)Alkyl, Phenyl oder substituiertes Phenyl,
- X: Halogen, vorzugsweise Chlor, Brom oder Iod, und
- n: null oder eins , insbesondere eins
bedeuten,
die als Ausgangsstoffe zur Herstellung einer Vielzahl biologisch aktiver Substanzen geeignet sind.

In der Formel (I) können die Kohlenstoffketten in den genannten Resten, wie gegebenenfalls substituierten Alkyl-, Alkoxy-, und anderen Resten, jeweils geradkettig oder verzweigt sein. Alkylreste, auch in zusammengesetzten Bedeutungen wie Alkoxy, Haloalkyl usw. bedeuten beispielsweise Methyl-, Ethyl-, n- oder i-Propyl, n-, i-, t- oder 2-Butyl usw. Alkenyl- und Alkinylreste haben die Bedeutung der den Alkylresten entsprechenden möglichen ungesättigten Reste, wie z. B. 2-Propenyl, 2- oder 3-Butenyl, 2-Propinyl, 2- oder 3-Butinyl. Halogen bedeutet Fluor, Chlor, Brom oder Iod. Aryl bedeutet einen aromatischen Rest, z. B. Phenyl, Naphthyl oder Fluorenyl, vorzugsweise Phenyl. Substituiertes Aryl oder Phenyl bedeutet z. B. Aryl bzw. Phenyl, das durch einen oder mehrere, vorzugsweise einen bis drei Reste aus der Gruppe Halogen, (C₁-C₄)Alkyl, (C₁-C₄)Alkoxy, (C₁-C₄)Thioalkyl, [(C₁-C₄)Alkyl]-carbonyl, [(C₁-C₄)Alkoxy]-carbonyl, [(C₁-C₄)Alkyl]-carbonyloxy, Carbonamid, [(C₁-C₄)Alkyl]-carbonylamino, [(C₁-C₄)Alkyl]aminocarbonyl, Di-[(C₁-C₄)Alkyl]-aminocarbonyl und Nitro substituiert ist. Substituiertes Aryl ist vorzugsweise Phenyl, das durch einen oder mehrere, vorzugsweise einen bis drei Reste aus der Gruppe Halogen, (C₁-C₄)Alkyl, (C₁-C₄)Alkoxy und Nitro substituiert ist.

Beispiele für die bei R₁ genannten Reste mit Carbonylgruppen sind Acetyl, Methoxycarbonyl, Ethoxycarbonyl, *tert*-Butoxycarbonyl, Benzyloxycarbonyl, Fluorenylmethoxycarbonyl und Naphthylmethoxycarbonyl.

Manche Verbindungen der Formel (I) können neben dem gezeigten Asymmetriezentrum ein oder mehrere asymmetrische C-Atome oder auch Doppelbindungen enthalten, die in der allgemeinen Formel (I) nicht gesondert angegeben sind. Die durch ihre spezifische Raumform definierten möglichen Stereoisomeren, wie Enantiomere, Diastereomere, Z- und E-Isomere sind jedoch alle von der Formel (I) umfaßt und können gegebenenfalls aus Gemischen der Stereoisomeren nach üblichen Methoden erhalten oder auch durch stereoselektive Reaktionen in Kombination mit dem Einsatz von stereochemisch reinen Ausgangsstoffen hergestellt werden.

Bevorzugte Verbindungen der genannten Formel I sind solche, worin
- R₁: 1-Hydroxy-(C₁-C₆)-Alkyl, Methansulfonyl, Benzolsulfonyl, o-, m- oder p-Toluolsulfonyl oder eine Gruppe der Formel R₂-CO-,
- R₂: (C₁-C₁₁)Alkyl, (C₆-C₁₂)Aryl oder substituiertes (C₆-C₁₂)Aryl, (C₁-C₄)Alkoxy, das unsubstituiert oder durch ein oder mehrere Reste, vorzugsweise einen Rest aus der Gruppe Phenyl und substituiertes Phenyl substituiert ist,
- X: Chlor, Brom oder Iod und
- n: null oder eins,
bedeuten.

Besonders bevorzugt sind Verbindungen der Formel I, worin
- R₁: 1-Hydroxy-(C₁-C₄)-Alkyl, Methansulfonyl, Benzolsulfonyl, p-Toluolsulfonyl, Methoxycarbonyl, Ethoxycarbonyl, tert.-Butoxycarbonyl oder Benzyloxycarbonyl
bedeutet.

Besonders bevorzugt sind auch solche Verbindungen der Formel I, welche eine Kombination der obengenannten bevorzugten Merkmale aufweisen.

Die optisch aktiven Verbindungen der Formel umfassen die (2S) bzw. (2R)-Verbindungen der Formeln I_{S} und I_{R}, 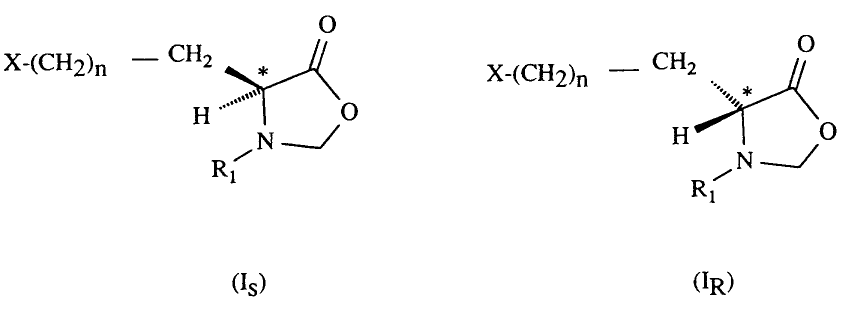
worin R₁, X und n die obengenannte Bedeutung haben.

Besonders bevorzugt sind optisch aktive (2S)-Verbindungen der Formel I_{S} oder Gemische der Isomeren I_{S} und I_{R} mit einem Gehalt von mehr als 80%, vorzugsweise mehr als 90% an der Verbindung der Formel I_{S}.

Zahlreiche Synthesen biologisch aktiver Wirkstoffe über vorwiegend acyclische ω-Halogen-2-amino-alkancarbonsäure-derivate sind bereits bekannt. Solche Verbindungen finden beispielsweise Verwendung bei der Synthese cystathioninhaltiger Peptide (Coll. Czech. Chem. Comm. **1967**, *32*, 2485), bei der Synthese von optisch aktiver Azetidincarbonsäure (Chem. Lett. **1973**, 5), bei der Herstellung modifizierter t-RNA (Chem. Ber. **1976**, *109*, 82), bei der Synthese von Süßstoffen (J. Agric. Food Chem. **1982**, *30*, 676) und vor allem bei der Synthese substituierter Aminosäuren (Tetrahedron **1985**, *41*, 1833) wie zum Beispiel L-Canalin (Lieb. Ann. Chem. **1986**, 287), L-Selenomethionin (Tetrahedron **1986**, 4983) oder Aminocyclopropancarbonsäure (Synthesis **1987**, 298). Ferner können sie zur Synthese des Keimhemmstoffs Discadenin (Synthesis **1988**, 240) sowie zur Herstellung von Antibiotica aus der Gruppe der Norcardicine (J. Am. Chem. Soc. **1990**, *112*, 760) verwendet werden.

Cyclische Derivate von ω-Halogen-2-aminoalkancarbonsäuren der obengenannten Formel I wurden dabei bisher jedoch nicht eingesetzt. In J. Chem. Soc. Chem. Comm. **1984**, 1298 ist die Herstellung einer Verbindung der Formel I, worin R₁ = Benzyloxycarbonyl, X = Br und n = 1 ist, beschrieben, wobei als Ausgangsmaterial das schlecht zugängliche bzw. teure N-Hydroxypyridin-2-thion verwendet wird. Die optische Ausbeute der beschriebenen Verbindung (Formel I, R₁ = Benzyloxycarbonyl, X = Br, n = 1) ist aufgrund des erhaltenen Drehwerts als sehr schlecht anzusehen. Die dabei erhaltene chemische Ausbeute von 73 % der Theorie ist für eine technische Nutzung ebenfalls zu gering.

Es wurde nun gefunden, daß die cyclischen Verbindungen der Formel I weitere wertvolle Synthone darstellen und besonders gut zur Herstellung bestimmter, weiter unten näher definierter optisch aktiver phosphorhaltiger α-Aminosäuren geeignet sind.

Ein Gegenstand der Erfindung sind deshalb optisch aktive, cyclische ω-Halogen-2-amino-alkancarbonsäure-derivate der genannten Formel I oder deren Gemische, worin R₁, X und n die bei Formel I genannte Bedeutung haben, ausgenommen die bekannte Verbindung der Formel I, worin R₁ = Benzyloxycarbonyl, X = Br und n = 1 ist.

Um das synthetische Potential der optisch aktiven, cyclischen ω-Halogen-2-aminoalkancarbonsäurederivate der Formel I voll ausschöpfen zu können, wird ein Verfahren benötigt, das von preiswerten, einfach zugänglichen Ausgangsmaterialien ausgeht und in hoher chemischer und optischer Ausbeute die gewünschten Verbindungen der allgemeinen Formel I liefert.

Weiterer Gegenstand der Erfindung ist ein solches Verfahren zur enantioselektiven Synthese der neuen und bekannten, optisch aktiven ω-Halogen-2-aminoalkancarbonsäure-derivaten der genannten Formel I, worin R₁, X und n die bei Formel I genannte Bedeutung haben, dadurch gekennzeichnet, daß man
a) im Falle der Verbindungen der genannten Formel I_{S} ein optisch aktives Diacylperoxid der allgemeinen Formel II_{S} (siehe Gleichung 1), worin R₁ und n die gleiche Bedeutung wie in Formel I besitzen, 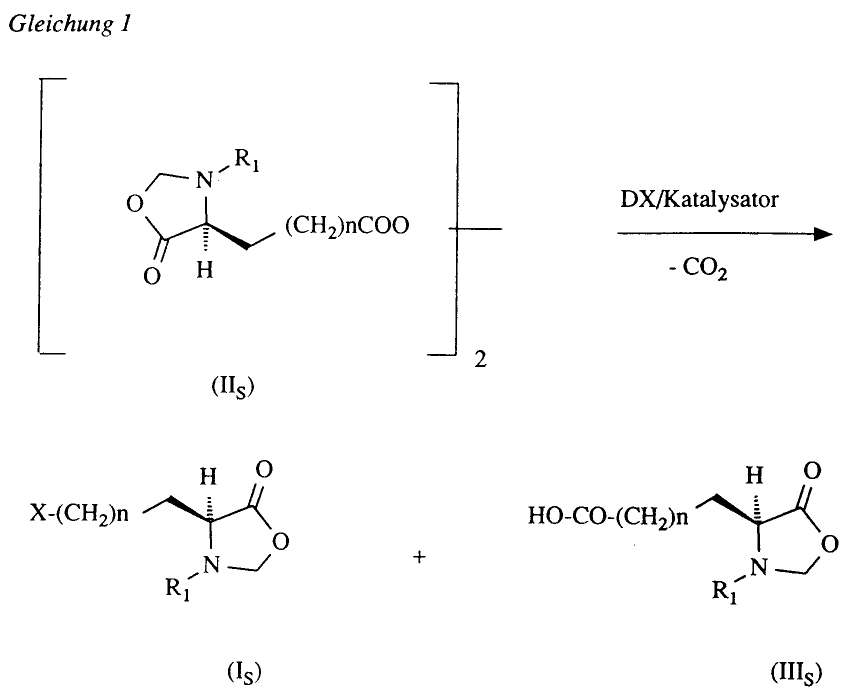 in einem nitrilgruppenhaltigen Lösungsmittel in Gegenwart eines kupferhaltigen Katalysators mit einem Halogenierungsmittel DX umsetzt und die Verbindung der Formel I_{S} aus dem entstehenden Gemisch der Verbindungen I_{S} und III_{S} (siehe Gleichung 1) isoliert oder
b) im Falle der Verbindungen der genannten Formel I_{R} oder Gemischen der Verbindungen der Formeln I_{R} und I_{S} ein optisch aktives Diacylperoxid II_{R}, das der Verbindung der unter a) genannten Formel II_{S} entspricht, jedoch anstelle der (S)-Konfiguration am jeweiligen asymmetrischen C-Atom die (R)-Konfiguration hat, bzw. ein entsprechendes Gemisch der Diacylperoxide II_{R} und II_{S} einsetzt und wie unter a) umsetzt und die Verbindung der Formel I_{R} bzw. das Gemisch der Verbindungen der Formel I_{R} und I_{S} isoliert.

Die bei der Umsetzung stattfindende Reaktion entspricht formal dem Hunsdiecker-Abbau und wurde bisher nur für unfunktionalisierte Diacylperoxide beschrieben (Lieb. Ann. Chem. **1961**, *649*,1; J. Am. Chem. Soc. **1965**, *87*, 1508).

Die Umsetzung der Acylperoxide der allgemeinen Formel II_{S} bzw. II_{R} wird vorzugsweise in einem Temperaturbereich von -30 bis +100 °C, insbesondere -5 bis +80 °C vorgenommen, in jedem Falle jedoch vorzugsweise bei einer Temperatur, die unterhalb der Zersetzungstemperatur der jeweiligen Verbindung liegt. Die Temperatur der unkatalysierten, thermischen Zersetzung kann durch bekannte Verfahren, wie zum Beispiel durch eine Differentialthermoanalyse ermittelt werden. Um der Gefahr einer spontanen Zersetzung vorzubeugen ist es außerdem ratsam, die Schlagempfindlichkeit des jeweils verwendeten Ausgangsmaterials zu überprüfen.

Als Lösungsmittel werden vor allem organische Nitrile verwendet. Beispiele hierfür sind aliphatische Mono- oder Dinitrile, wie Acetonitril, Propionitril und auch Octandisäuredinitril, oder aromatische Nitrile, wie Benzonitril und substituierte Benzonitrile.

Ebenso können Gemische aus Nitrilen und anderen Lösungsmitteln, beispielsweise Carbonsäuren eingesetzt werden, um die Löslichkeit des jeweils verwendeten Kupferkatalysators zu erhöhen. Beispiele fuhr einsetzbare Carbonsäuren sind Essigsäure, Chloressigsäure oder Propionsäure.

Als Halogenierungsmittel DX sind z. B. die elementaren Halogene, wie Chlor, Brom und Iod, Phosphor-V-halogenide oder Kupferhalogenide geeignet.

Als Katalysatoren eignen sich Kupferverbindungen in allen Wertigkeitsstufen, z. B. Kupfer-I-oxid und Kupferhalogenide wie Kupfer-I-bromid, Kupfer-II-bromid und Kupfer-I-iodid.

So dienen im Falle von X=Br z. B. elementares Brom oder Phosphor-V-bromid als Halogenierungsmittel DX, im Fall von X=Cl z. B. Phosphor-V-chlorid, wobei als Katalysator in diesen Fällen Kupfer-I-oxid bevorzugt verwendet wird.

Bei Verwendung der elementaren Halogene können als Katalysatoren die entsprechenden Kupferhalogenide eingesetzt werden, so zum Beispiel die Kombination Br₂/CuBr₂.

Im Fall X= Br und X= Iod besteht eine besonders bevorzugte Variante des erfindungsgemäßen Verfahrens darin, als Halogenierungsmittel und zugleich als Katalysator Kupferhalogenide einzusetzen. So können zur Herstellung von Verbindungen der allgemeinen Formel I vorteilhafterweise Kupfer-I-bromid, Kupfer-II-bromid oder Kupfer-I-iodid eingesetzt werden. Auch für den Fall X= Cl lassen sich Kupfer-I-chlorid und Kupfer-II-chlorid einsetzen.

Die verwendete Katalysatormenge kann in weiten Grenzen variiert werden, jedoch sollte bei Verwendung der Kupferhalogenide sichergestellt sein, daß zumindest die stöchiometrisch notwendige Menge Halogenid im System anwesend ist.

Die Reaktionsdauer ist abhängig von der Reaktionstemperatur und liegt zwischen 0.5 und 30 Stunden.

Alle Reaktionen werden zweckmäßig unter Sauerstoffausschluß durchgeführt, daß heißt, unter einer Schutzgasatmosphäre oder im Vakuum. Als Schutzgase kommen zum Beispiel Stickstoff oder Argon in Frage.

Die Isolierung der Verbindung der Formel I aus dem Reaktionsgemisch kann nach üblichen Methoden erfolgen, wie zum Beispiel durch Säure-Base-Trennung oder Chromatographie des Gemisches, das die Verbindungen der Formeln I (I_{S} bzw. I_{R}) und III (III_{S} bzw. III_{R}) enthält.

Zur Auftrennung der Verbindungen der Formeln I_{S} und III_{S} (bzw. I_{R} und III_{R}) wird beispielsweise das während der Reaktion verwendete Lösungsmittel vollständig abdestilliert und der Rückstand in einem nicht mit Wasser mischbaren Lösungsmittel, wie zum Beispiel Toluol, Xylol, Dichlorbenzol, Ethylacetat oder Dichlormethan oder Mischungen aus diesen Lösungsmitteln aufgenommen. Zur Abtrennung der Carbonsäurekomponente III wird die organische Phase dann mit verdünnter wäßriger Natronlauge. verdünnter Hydrogencarbonatlösung, Hydrogenphosphatpufferlösung oder einer anderen basischen Lösung gewaschen, wobei der pH-Wert von 8.5 vorzugsweise nicht überschritten wird. Die auf diese Weise in der organischen Phase enthaltene rohe Verbindung der Formel I kann durch Abdestillieren des Lösungsmittels, in der Regel unter reduziertem Druck, isoliert und, falls dies gewünscht wird, durch bekannte Methoden wie Umkristallisation oder Chromatographie weiter gereinigt werden.

Nach dem Ansäuern der alkalischen, wässrigen Phase kann man aus dieser die Carbonsäurekomponente der Formel III mit einem organischen Lösungsmittel extrahieren und in üblicher Weise isolieren. Falls es gewünscht wird läßt sich die so zurückgewonnene Carbonsäurekomponente wieder zur Herstellung des als Ausgangsmaterial benötigten Diacylperoxids der Formel II einsetzen.

Diacylperoxide der allgemeinen Formel II sind neu und daher ebenfalls Gegenstand der vorliegenden Erfindung.

Die als Ausgangsmaterial benötigten Diacylperoxide II (II_{S} bzw. II_{R}) können aus den Carbonsäurechloriden der Formel IIIa (IIIa_{S} bzw. IIIa_{R}) durch Umsetzung mit Wasserstoffperoxid gewonnen werden (siehe Gleichung 2, dort nur für die S-Formen wiedergegeben). 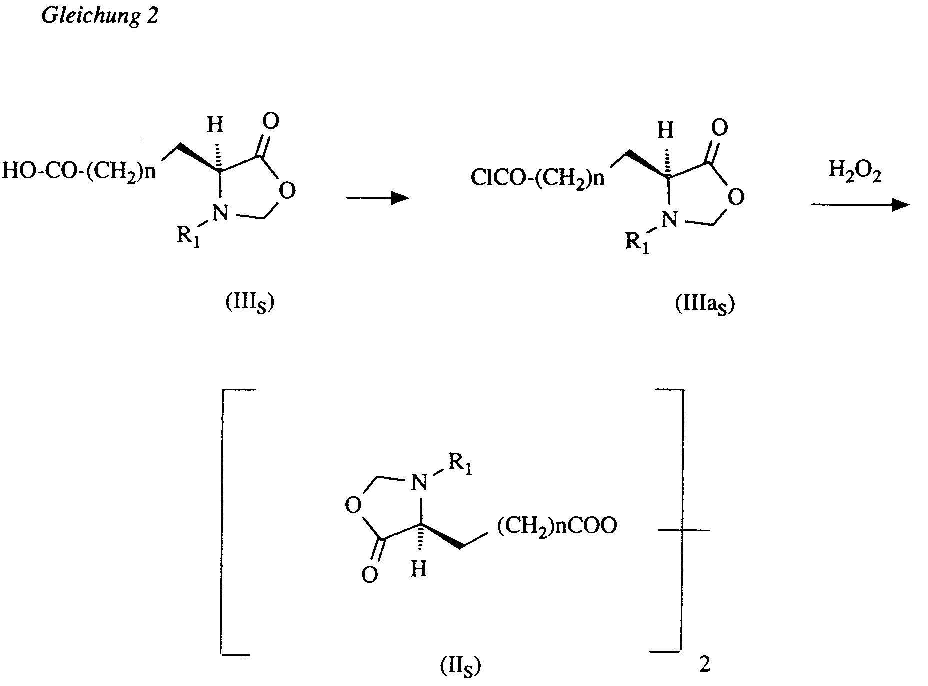
Die Umsetzung mit H₂O₂ wird beispielsweise in einem organischen oder wäßrigorganischen Lösungsmittel in Gegenwart einer Base durchgeführt. Geeignete Lösungsmittel sind dafür organische Lösungsmittel aus der Gruppe der aliphatischen oder aromatischen, gegebenenfalls halogenierten Kohlenwasserstoffe, Ether wie Diethylether oder Tetrahydrofuran und Ester wie Ethylacetat. Als Basen sind anorganische und organische Basen geeignet, beispielsweise organische Basen aus der Gruppe der tertiären organischen Amine wie Pyridin, Triethylamin und 1,8-Diazabicyclo[5.4.0]undec-7-en . Die Reaktionstemperatur ist in der Regel bei -20 bis +20°, vorzugsweise bei 0 bis 10°C.

Die Herstellung der Carbonsäurechloride der Formel IIIa aus den freien Carbonsäuren der Formel III sowie die Herstellung der Carbonsäuren der Formel III können nach oder analog bekannten Methoden erfolgen (Synthesis **1989**, 542; Tetrahedron **1986**, *42*, 6551; Rec. Trav. Chim. **1975**, *94*, 182; Coll. Czech. Chem. Comm. **1963**, *28*, 2941).

Die Verbindungen der Formel III, insbesondere III_{S}, können in bekannter Weise aus Asparaginsäure und Glutaminsäure, insbesondere L-Asparaginsäure bzw. L-Glutaminsäure erhalten werden (Lit.: Chem. Pharm. Bull. **1969**, 1679; Synthesis **1989**, 542; Tetrahedron **1986**, 6551; J. Chem. Soc. Chem. Comm. **1989**, 423; Chemische Berichte **95**, 1009 (1962)). Es ist ein besonderer Vorteil der oben und im folgenden beschriebenen erfindungsgemäßen Herstellungsverfahren, daß die in großen Mengen verfügbaren und preiswerten Aminosäuren L-Asparaginsäure und L-Glutaminsäure als Ausgangsmaterialien zur Herstellung optisch aktiver Synthone und Wirkstoffe verwendet werden können.

Gegenstand der Erfindung ist weiterhin die Verwendung der auf diese Weise erhaltenen Verbindungen der allgemeinen Formel I zur Synthese von Derivaten optisch aktiver, phosphorhaltiger α-Aminosäuren der allgemeinen Formel V. Das erfindungsgemäße Verfahren zur Herstellung der Verbindungen der Formel V gehört zum Typ einer Arbusov-Reaktion und ist in Gleichung 3 am Beispiel der Verbindungen mit (2S)-Konfiguration der Formeln I_{S} und V_{S} dargestellt. 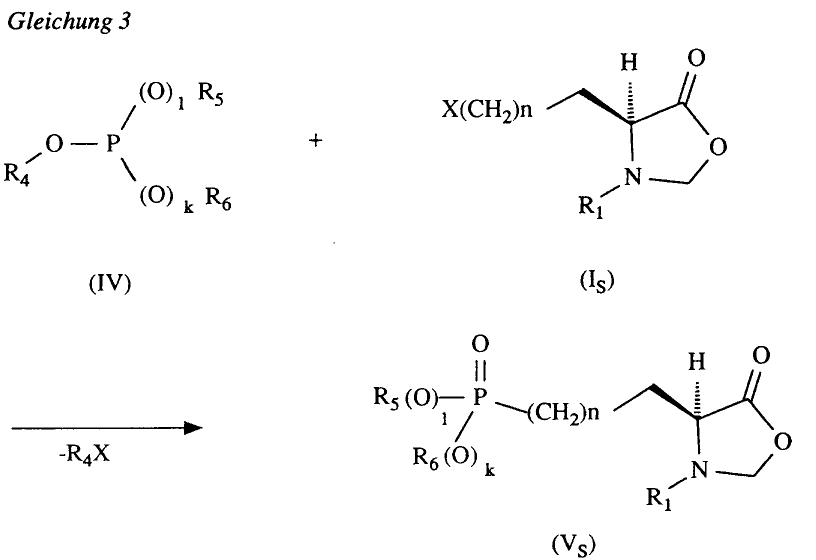
In den Formeln I, IV und V (bzw. I_{S}, I_{R}, IV_{S}, IV_{R}, V_{S}, V_{R}) bedeuten
- R₄: Trialkylsilyl, (C₁-C₈)-Alkyl, (C₃-C₈)-Cycloalkyl, (C₆-C₁₃)-Aryl oder substituiertes (C₆-C₁₃)-Aryl,
- R₅, R₆: unabhängig voneinander (C₁-C₈)-Alkyl, (C₃-C₈)-Cycloalkyl, (C₆-C₁₃)-Aryl oder substituiertes (C₆-C₁₃)-Aryl,
- k: null oder eins, vorzugsweise eins und
- l: null oder eins, vorzugsweise null;
- R₁, X und n: haben die weiter oben bereits genannte Bedeutung.

Trialkylsilyl ist in der obigen Definition von R₄ vorzugsweise Tri-(C₁-C₆-alkyl)-silyl, insbesondere Trimethylsilyl, Triethylsilyl, Tributylsilyl und *tert*-Butyldimethylsilyl zu verstehen.

Die nach dem erfindungsgemäßen Verfahren (vgl. Gleichung 3) erhältlichen cyclischen Derivate optisch aktiver, phosphorhaltiger α-Aminosäuren der allgemeinen Formel V sind teilweise in der DE-OS-3817956 (ZA 89/4012) beschrieben und können als Zwischenprodukte zur der Herstellung der freien Aminosäuren der Formel VI und ihrer Peptide eingesetzt werden (vgl. Gleichung 4). Die Verbindungen der Formel VI und zahlreiche ihrer Peptide besitzen herbizide, fungizide und antivirale Eigenschaften (siehe Bull. Chem. Soc. Japan **1987**, *60*, 1761; DE-OS 2 856 260 (GB-A-2011416); Sci. Rep. Meiji Seika Kaisha **1973**, *13*, 34; Biochem. **1964**, *3*, 991).

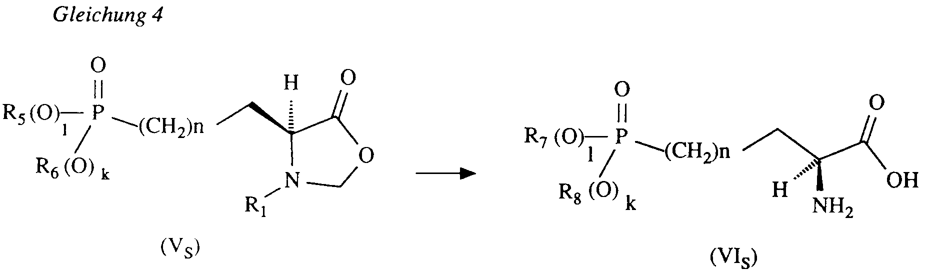

In der Formel VI (bzw. VI_{S} oder VI_{R}) sind
R₇ = R₅ oder, wenn l = 1 und k = 1 sind, R₅ oder Wasserstoff und
R₈ = R₆ oder, wenn l = 1 und k = 1 sind, R₆ oder Wasserstoff, und
R₁, n, k und l haben die bereits bei Formel I bzw. Formel IV genannten Bedeutungen.

Bei den Verbindungen der Formel (VI) bzw. (VI_{S}) kommt der Aminosäure 4-[Hydroxy(methyl)phosphinoyl]-homoalanin (VI) bzw. vorzugsweise (2S)-4-[Hydroxy(methyl)-phosphinoyl]-homoalanin (VI_{S}), letzteres im folgenden kurz L-Phosphinothricin oder kurz L-PTC genannt (siehe nachstehende Formel), besondere Bedeutung zu, die in freier Form oder in Form ihrer Salze die aktive Substanz des Herbizids Glufosinate (D,L-PTC) darstellt (vgl. "The Pesticide Manual", 9. Aufl. 1991, 458): 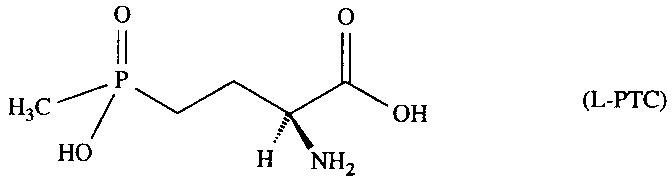
Das erfindungsgemäße Verfahren erlaubt somit die Herstellung von L-Phosphinothricin und anderen phosphorhaltigen L-Aminosäuren ausgehend von den gut zugänglichen Aminosäuren L-Glutaminsäure bzw. L-Asparaginsäure.

Die Umsetzung von Verbindungen der allgemeinen Formel I mit Phosphorigsäure-, Phosphonigsäure- oder Phosphinigsäurederivaten der allgemeinen Formel IV führt, wie in Gleichung 3 dargestellt, in einer Arbusov-Reaktion (Chem. Rev. **1981**, *81*, 415) zu den cyclischen Derivaten optisch aktiver, phosphorhaltiger α-Aminosäuren V, die wie in der DE-OS 3 817 956 (ZA 89/4012) beschrieben, weiter derivatisiert werden können. Verwendet man als Phosphorkomponente beispielsweise Diester der methanphosphonigen Säure, so erhält man durch Reaktion mit einer Verbindung der Formel I_{S} Verbindungen der Formel Va_{S}, die auf bekannte Weise in L-PTC umgewandelt werden können (DE-OS 3 817 956 (ZA 89/4012)). 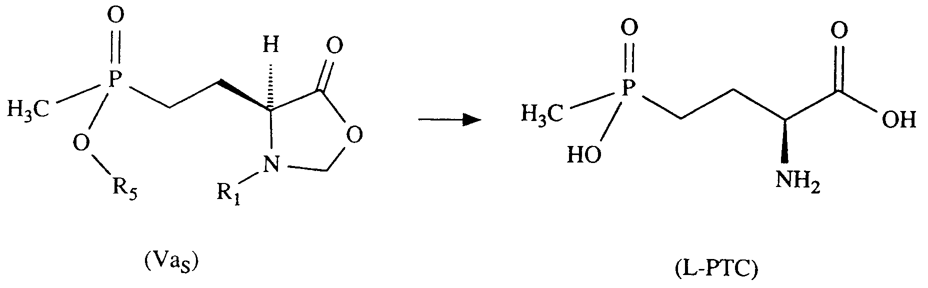
Ausgehend von L-Glutaminsäure laßt sich auf diese Weise L-Phosphinothricin in einer Enantiomerenreinheit von mehr als 94% ee gewinnen.

An den nachfolgenden Beispiele wird die Erfindung näher erläutert; sie ist jedoch nicht auf die beispielhaft gezeigten Verfahrensweisen beschränkt. In den Beispielen beziehen sich Prozentangaben auf das Gewicht, sofern nichts anderes angegeben ist.

### Beispiel 1

### Bis-{3-[(4S)-3-benzyloxycarbonyl-5-oxo-1.3-oxazolidin-4-yl]-propionyl}-peroxid

15.9 ml (15.6 g, 0.197 mol) Pyridin werden in 250 ml Diethylether gelöst, auf 0 °C abgekühlt und mit 14.5 g Wasserstoffperoxid-Harnstoff-Komplex (enthält 35 % Wasserstoffperoxid, 0.054mol) versetzt. Bei 0-5 °C werden portionsweise 30.7 g (0.098 mol) 3-[(4S)-3-Benzyloxycarbonyl-5-oxo-1.3-oxazolidin-4-yl]-propionsäurechlorid (hergestellt nach: Rec. Trav. Chim. **1975**, *94*, 182) eingetragen. Die Mischung wird 5 Stunden unter Eiskühlung gerührt, dann werden 350 ml Ethylacetat zugegeben. Die Reaktionsmischung wird einmal mit 250 ml 10%-iger Schwefelsäure, zweimal mit mit je 150 ml gesättigter Natriumhydrogencarbonatlösung und einmal mit Wasser gewaschen. Die organische Phase wird abgetrennt, über Magnesiumsulfat getrocknet und unter reduziertem Druck (Anlegen von Hochvakuum) bei Temperaturen unter 30 °C eingeengt. Der Rückstand wird durch Chromatographie an Kieselgel (Laufmittel: Toluol/Ethylacetat) gereinigt. Man erhält 13.9 g (48.5 % der Theorie) Bis-{3-[(4S)-3-benzyloxycarbonyl-5-oxo-1.3-oxazolidin-4-yl]-propionyl}-peroxid als weißen Feststoff. Analytische Daten: Peroxidgehalt jodometrisch: 100%;
¹H NMR (100 MHz,CDCl₃) 7.37 (s, 10, C₆**H₅**), 5.54 (d, 2, J=5 Hz, NC**H₂**O), 5.23 (d, 2, J= 5 Hz, NC**H₂**O), 5.19 (s, 4, OC**H****₂**-C₆H₅), 4.38 (t, 2, J= 5.5 Hz, C**H**(NCO)COO), 2.72-2.08 (m, 8, OCC**H****₂**C**H****₂**CH);
¹³C NMR (75 MHz,CDCl₃) 171.240, 167.712, 153.074, 135.178, 128.735, 128.718, 128.434, 77.877, 68.333, 53.815, 25.868, 25.504;

| | | | | |
|---|---|---|---|---|
| C₂₈H₂₈N₂O₁₂ (584.54): | Ber. | C, 57.53; | H, 4.83; | N, 4.79 |
| | Gef. | C, 57.80; | H, 4.90; | N, 4.80. |

### Beispiel 2

### Bis-{3-[(4S)-3-benzyloxycarbonyl-5-oxo-1.3-oxazolidin-4-yl]-acetyl}-peroxid

Ausgehend von 3-[(4S)-3-Benzyloxycarbonyl-5-oxo-1.3-oxazolidin-4-yl]-essigsäurechlorid (hergestellt nach: Tetrahedron **1986**, *42*, 6551) erhält man analog zu Beispiel 1 Bis-{3-[(4S)-3-benzyloxycarbonyl-5-oxo-1.3-oxazolidin-4-yl]-acetyl}-peroxid in 56.8% der Theorie Ausbeute. Analytische Daten:
- ¹H NMR (100 MHz,CDCl₃): 7.35 (s, 10, C₆**H₅**), 5.45 (d, 2, J= 4.5 Hz, NC**H₂**O), 5.28 (d, 2, J= 4.5 Hz, NC**H₂**O), 5.18 (s, 4, OC**H****₂**C₆H₅), 4.45 (t, 2, J= 4.5 Hz, C**H**(NCO)COO), 3.49-2.85 (m, 4, OCC**H****₂**CH).

### Beispiel 3

### Bis-{3-[(4S)-3-toluolsulfonyl-5-oxo-1.3-oxazolidin-4-yl]-propionyl}-peroxid

Ausgehend von 3-[(4S)-3-p-Toluolsulfonyl-5-oxo-1.3-oxazolidin-4-yl]-propionsäurechlorid (hergestellt nach: Coll. Czech. Chem. Comm. **1963**, *28*, 2941 ) erhält man analog zu Beispiel 1 Bis-{3-[(4S)-3-toluolsulfonyl-5-oxo-1.3-oxazolidin-4-yl]**-**propionyl}-peroxid in 30.2% der theoretischen Ausbeute. Analytische Daten:
¹H NMR (100 MHz,CDCl₃) 7.71 (d, 4, J= 7.75 Hz, C₆**H₅**), 7.35 (d, 4, J= 7.75 Hz, C₆**H₅**), 5.49 (d, 2, J= 7.25 Hz, NC**H₂**O), 5.24 (d, 2, J= 7.25 Hz, NC**H₂**O), 4.12 (m, 2, C**H**(NCO)COO), 2.92-2.57 (m, 4, OCC**H****₂**CH₂CH), 2.53-1.92 (m, 4, OCCH₂C**H****₂**CH), 2.43 (s, 6, C₆H₅C**H₃**);

| | | | | |
|---|---|---|---|---|
| C₂₆H₂₈N₂O₁₂S₂ (624.65): | Ber. | C, 49.99; | H, 4.52; | N, 4.48 |
| | Gef. | C, 49.80; | H, 4.80; | N, 4.50. |

### Beispiel 4

### (4S)-3-Benzyloxycarbonyl-4-(2-chlorethyl)-1.3-oxazolidin-5-on

3.36 g (23.5 mmol) Kupfer-I-oxid werden unter Argonatmosphäre in 280 ml einer 1:1-Mischung aus Acetonitril und Essigsäure suspendiert und 10 min bei Raumtemperatur gerührt. Die entstandene klare Lösung wird auf 50 °C erhitzt und nach Zugabe von 4.89 g (23.5 mmol) Phosphor-V-chlorid tropfenweise mit einer Lösung von 13.60 g (23.3 mmol) Bis-{3-[(4S)-3-benzyloxycarbonyl-5-oxo-1.3-oxazolidin-4-yl]-propionyl}-peroxid (erhalten aus Beispiel 1) in 75 ml Acetonitril versetzt. Es wird noch 5.5 Stunden bei 50 °C und 18 Stunden bei Raumtemperatur gerührt, dann wird das Lösungsmittel unter reduziertem Druck (Anlegen von Hockvakuum) vollständig abdestilliert und der Rückstand in 600 ml Ethylacetat aufgenommen. Die organische Phase wird einmal mit 400 ml 0.25 N Salzsäure und zweimal mit je 400 ml Wasser gewaschen, über Magnesiumsulfat getrocknet und vollständig eingeengt. Man erhält 12.7 g eines rötlichen Oels, das laut ¹H-NMR-Spektrum zu 47 % aus (4S)-3-Benzyloxycarbonyl-4-(2-chlorethyl)-1.3-oxazolidin-5-on und zu 53 % aus 3-[(4S)-3-Benzyloxycarbonyl-5-oxo-1.3-oxazolidin-4-yl]-propionsäure besteht. Man löst dieses Oel in 150 ml Toluol, wäscht die organische Phase einmal mit 75 ml und einmal mit 40 ml gesättigter Natriumhydrogencarbonatlösung, trocknet über Magnesiumsulfat und engt ein. Nach Chromatographie an Kieselgel (Laufmittel: Toluol/Ethylacetat) erhält man 4.9 g (74.1% der Theorie) (4S)-3-Benzyloxycarbonyl-4-(2-chlorethyl)-1.3-oxazolidin-5-on als weißen Feststoff. Analytische Daten:
Fp 75-77 °C;
[α]_{D}²³= 107.30 ° (c= 0.481 CHCl₃);
¹H NMR (100 MHz,CDCl₃) 7.36 (s, 5, C₆**H₅**), 5.54 (d, 1, J= 5 Hz, NC**H₂**O), 5.27 (dd, 1, J= 5 Hz, 2 Hz, NC**H₂**O), 5.19 (m, 2, OC**H₂**C₆H₅), 4.44 (td, 1, J= 5.5 Hz,2 Hz, C**H**(NCO)COO), 3.63 (t, 2, J = 7 Hz, ClC**H****₂**CH₂), 2.53-2.25 (m, 2, ClCH₂C**H****₂**CH);
¹³C NMR (75 MHz,CDCl₃) 171.671, 152.977, 135.231, 128.758, 128.408, 77.924, 68.230, 52.467, 39.685, 33.209 ;

| | | | | | |
|---|---|---|---|---|---|
| C₁₃H₁₄NO₄Cl (283.71): | Ber. | C, 55.04; | H, 4.97; | N, 4.94; | Cl,12.50 |
| | Gef. | C, 55.50; | H, 5.10; | N, 4.80. | |

### Beispiel 5

### (4S)-3-Benzyloxycarbonyl-4-(2-bromoethyl)-1.3-oxazolidin-5-on

4.30 g (19.3 mmol) Kupfer-II-bromid werden unter Argonatmosphäre in 320 ml Acetonitril gelöst, auf 50 °C erhitzt und tropfenweise mit einer Lösung von 20.43 g (35 mmol) Bis-{3-[(4S)-3-benzyloxycarbonyl-5-oxo-1.3-oxazolidin-4-yl]-propionyl}-peroxid (aus Beispiel 1) in 90 ml Acetonitril versetzt. Die Mischung wird 5.5 Stunden bei 50 °C und 18 Stunden bei Raumtemperatur gerührt. Das Lösungsmittel wird unter reduziertem Druck (Anlegen von Hockvakuum) vollständig abdestilliert, der Rückstand in 500 ml Ethylacetat aufgenommen, einmal mit 500 ml 0.25 N Salzsäure und zweimal mit je 50 ml Wasser gewaschen. Die organische Phase wird über Magnesiumsulfat getrocknet und eingeengt. Man erhält 23.9 g eines halbkristallinen Feststoffs der laut ¹H-NMR-Spektrum zu 48% aus (4S)-3-Benzyloxycarbonyl-4-(2-bromethyl)-1.3-oxazolidin-5-on und zu 52% aus 3-[(4S)-3-Benzyloxycarbonyl-5-oxo-1.3-oxazolidin-4-yl]-propionsäure besteht. Dieser Feststoff wird in 350 ml Toluol gelöst, einmal mit 300 ml und einmal mit 200 ml gesättigter Natriumhydrogencarbonatlösung gewaschen, über Magnesiumsulfat getrocknet und im Vakuum eingeengt. Nach Chromatographie an Kieselgel (Laufmittel: Toluol/Ethylacetat) erhält man 10.3 g (89.6% der Theorie) (4S)-3-Benzyloxycarbonyl-4-(2-bromethyl)-1.3-oxazolidin-5-on als weißen Feststoff. Analytische Daten:
Fp 85 °C [Literaturwert aus J. Chem. Soc. Chem. Comm. **1984**, 1298: 66.5 °C];
[α]_{D}²³= 86 ° (c= 0.929, CH₃OH) [Literaturwert aus J. Chem. Soc. Chem. Comm. **1984**, 1298: [α]_{D}= 54 ° (c= 0.9, CH₃OH)];
¹H NMR (100 MHz,CDCl₃) 7.37 (s, 5, C₆**H₅**), 5.55 (d, 1, J= 5 Hz, NC**H₂**O), 5.26 (d, 1, J= 5 Hz, NC**H₂**O), 5.18 (s, 2, OC**H****₂**C₆H₅), 4.43 (t, 1, J= 5.5 Hz, C**H**(NCO)COO), 3.46 (t, 2, J = 7 Hz, BrC**H****₂**CH₂), 2.60-2.31 (m, 2, BrCH₂C**H****₂**CH);
¹³C NMR (75 MHz,CDCl₃) 171.466, 152.976, 135.190, 128.760, 128.453, 77.960, 68.262, 53.577, 33.568, 26.972;

| | | | | | |
|---|---|---|---|---|---|
| C₁₃H₁₄NO₄Br (328.17): | Ber. | C, 47.58; | H, 4.30; | N, 4.27; | Br,24.35 |
| | Gef. | C, 47.80; | H, 4.30; | N, 4.10; | Br,25.20. |

Ausgehend von Bis-{3-[(4S)-3-benzyloxycarbonyl-5-oxo-1.3-oxazolidin-4-yl]-propionyl}-peroxid (aus Beispiel 1) wird mit verschiedenen Halogendonor/Katalysatorkombinationen (4S)-3-Benzyloxycarbonyl-4-(2-bromethyl)-1.3-oxazolidin-5-on in folgenden Ausbeuten (% der Theorie) erhalten:

| | **Halogendonor** | **Katalysator** | **Lösungsmittel** | **Ausbeute** |
|---|---|---|---|---|
| Beispiel 6 | CuBr₂ | CuBr₂ | Benzonitril | 67.1% |
| Beispiel 7 | CuBr | CuBr | Acetonitril | 60.0% |
| Beispiel 8 | PBr₅ | Cu₂O | Acetonitril/Essigsäure | 75.0% |
| Beispiel 9 | Br₂ | CuBr₂ | Acetonitril | 78.2% |

### Beispiel 10

### (4S)-3-Benzyloxycarbonyl-4-(2-iodethyl)-1.3-oxazolidin-5-on

1.05 g (5.5 mmol) Kupfer-I-iodid werden unter Argonatmosphäre in 50 ml Acetonitril gelöst, auf 50 °C erhitzt und tropfenweise mit einer Lösung von 2.92 g (5 mmol) Bis-{3-[(4S)-3-benzyloxycarbonyl-5-oxo-1.3-oxazolidin-4-yl]-propionyl}-peroxid (aus Beispiel 1) in 14 ml Acetonitril versetzt. Die Mischung wird 5.5 Stunden bei 50 °C und 18 Stunden bei Raumtemperatur gerührt. Das Lösungsmittel wird unter Anlegen von Hochvakuum vollständig abdestilliert, der Rückstand in 100 ml Ethylacetat aufgenommen, einmal mit 75 ml 0.25 N Salzsäure und zweimal mit je 75 ml Wasser gewaschen. Die organische Phase wird über Magnesiumsulfat getrocknet und eingeengt. Man erhält 3.1 g eines braunen Oels das laut ¹H-NMR-Spektrum zu 34% aus (4S)-3-Benzyloxycarbonyl-4-(2-iodethyl)-1.3-oxazolidin-5-on und zu 66% aus 3-[(4S)-3-Benzyloxycarbonyl-5-oxo-1.3-oxazolidin-4-yl]-propionsäure besteht. Dieses Oel wird in 35 ml Toluol gelöst, einmal mit 20 ml gesättigter Natriumhydrogencarbonatlösung gewaschen, über Magnesiumsulfat getrocknet und im Vakuum eingeengt. Umkristallisation aus Methanol führt zu 0.95 g (50.6% der Theorie) (4S)-3-Benzyloxycarbonyl-4-(2-iodethyl)-1.3-oxazolidin-5-on als hellbraunen Feststoff. Analytische Daten:
Fp 79-80 °C
[α]_{D}²³= 80.5 ° (c= 0.539, CHCl₃);
- ¹H NMR (100 MHz,CDCl₃): 7.37 (s, 5, C₆**H₅**), 5.55 (d, 1, J= 5 Hz, NC**H₂**O), 5.24 (d, 1, J= 5 Hz, NC**H₂**O), 5.18 (s, 2, OC**H****₂**C₆H₅), 4.37 (t, 1, J= 5.5 Hz, C**H**(NCO)COO), 3.21 (t, 2, J = 7 Hz, JC**H****₂**CH₂), 2.44 (q, 2, JCH₂C**H₂**CH)

### Beispiel 11

### (4S)-3-Benzyloxycarbonyl-4-(2-brommethyl)-1.3-oxazolidin-5-on

1.40 g (6.23 mmol) Kupfer-II-bromid werden unter Argonatmosphäre in 100 ml Acetonitril gelöst, auf 50 °C erhitzt und tropfenweise mit einer Lösung von 6.40 g (11.5 mmol) Bis-{3-[(4S)-3-benzyloxycarbonyl-5-oxo-1.3-oxazolidin-4-yl]-acetyl}-peroxid (aus Beispiel 2) in 35 ml Acetonitril versetzt. Die Mischung wird 4.5 Stunden bei 50 °C und 18 Stunden bei Raumtemperatur gerührt. Das Lösungsmittel wird im Hochvakuum vollständig abdestilliert, der Rückstand in 250 ml Ethylacetat aufgenommen, einmal mit 175 ml 0.25 N Salzsäure und einmal mit 175 ml Wasser gewaschen. Die organische Phase wird über Magnesiumsulfat getrocknet und eingeengt. Man erhält 15.5 g eines Oels das laut ¹H-NMR-Spektrum eine Mischung aus (4S)-3-Benzyloxycarbonyl-4-(2-brommethyl)-1.3-oxazolidin-5-on und 3-[(4S)-3-Benzyloxycarbonyl-5-oxo-1.3-oxazolidin-4-yl]-essigsäure darstellt. Dieses Oel wird in 170 ml Toluol gelöst, einmal mit 120 ml und einmal mit 60 ml gesättigter Natriumhydrogencarbonatlösung gewaschen, über Magnesiumsulfat getrocknet und im Vakuum eingeengt. Man erhält 2.85 g (78.9% der Theorie) (4S)-3-Benzyloxycarbonyl-4-(2-brommethyl)-1.3-oxazolidin-5-on als hellbraunen Feststoff. Analytische Daten:
- ¹H NMR (100 MHz,DMSO-*d₆*): 7.37 (m, 5, C₆**H₅**), 5.44 (d, 1, J= 3.75 Hz, NC**H₂**O), 5.32 (d, 1 , J= 3.75 Hz, NC**H₂**O), 5.20 (s, 2, OC**H****₂**C₆H₅), 4.88 (m, 1, C**H**(NCO)COO), 3.91 (m, 2,BrC**H₂**CH).

### Beispiel 12

### (4S)-3-Benzyloxycarbonyl-4-{2-[ethoxy(methyl)phosphinyl]-ethyl}-1.3-oxazolidin-5-on

5.30 g (16.1 mmol) (4S)-3-Benzyloxycarbonyl-4-(2-bromethyl)-1.3-oxazolidin-5-on (aus Beispiel 5) werden mit 4.40 g (32.3 mmol) Methanphosphonigsäurediethylester vermischt und unter Argon 6 Stunden auf 120 °C erhitzt. Der überschüssige Methanphosphonigsäurediethylester wird im Hochvakuum abdestilliert und der Rückstand durch Chromatographie an Kieselgel (Laufmittel: Dichlormethan/Acetonitril) gereinigt. Man erhält 4.64 g (81.1% der Theorie) (4S)-3-Benzyloxycarbonyl-4-{2-[ethoxy(methyl)phosphinyl]-ethyl}-1.3-oxazolidin-5-on als farbloses Oel. Analytische Daten:
[α]_{D}²³= 91.61 ° (c= 0.560, CHCl₃)
- ¹H NMR (100 MHz,CDCl₃): 7.37 (s, 5, C₆**H₅**), 5.53 (d, 1, J= 5 Hz, NC**H₂**O), 5.23 (d, 1, J= 5 Hz, NC**H₂**O), 5.18 (s, 2, OC**H****₂**C₆H₅), 4.37 (t, 1, J= 5.5 Hz, C**H**(NCO)COO), 4.01 (quin d, 2, J= 7 Hz, 1.2Hz, POC**H****₂**CH₃), 2.47-1.55 (m, 4, PC**H****₂**C**H****₂**CH), 1.42 (d, 3, J= 14 Hz, **H₃**CP(O)), 1.28 (td, 3, J= 7 Hz, 1.2 Hz, POCH₂C**H₃**);
- ³¹P NMR (121 MHz,CDCl₃): 52.863

Die so erhaltene Substanz ist identisch mit der in der DE-A-3817956 beschriebenen Verbindung.

### Beispiel 13

### (4S)-3-Benzyloxycarbonyl-4-[2-(diethoxyphosphino)ethyl]-1.3-oxazolidin-5-on

3.28 g (10 mmol) (4S)-3-Benzyloxycarbonyl-4-(2-bromethyl)-1.3-oxazolidin-5-on (aus Beispiel 5) werden mit 3.39 g (20 mmol) Phosphorigsäuretriethylester vermischt und unter Argon 18 Stunden auf 120 °C erhitzt. Der überschüssige Phosphorigsäuretriethylester wird unter Anlegen von Hochvakuum abdestilliert und der Rückstand durch Chromatographie an Kieselgel (Laufmittel: Dichlormethan/Acetonitril) gereinigt. Man erhält 1.34 g (34.8% der Theorie) (4S)-3-Benzyloxycarbonyl-4-[2-(diethoxyphosphinyl)ethyl]-1.3-oxazolidin-5-on als farbloses Oel. Analytische Daten:
[α]_{D}²³= 82.10 ° (c= 0.402, CHCl₃);
¹H NMR (100 MHz,CDCl₃) 7.37 (s, 5, C₆**H₅**), 5.53 (d, 1, J= 5 Hz, NC**H₂**O), 5.23 (d, 1, J= 5 Hz, NC**H₂**O), 5.18 (s, 2, OC**H****₂**C₆H₅), 4.35 (t, 1, J= 5.5 Hz, C**H**(NCO)COO), 4.07 (quin, 4, J= 7 Hz, POC**H****₂**CH₃), 2.45-1.43 (m, 4, PC**H****₂**C**H****₂**CH), 1.28 (t, 3, J= 7 Hz, POCH₂C**H₃**);
¹³C NMR (75 MHz,CDCl₃) 171.395, 152.906, 135.230, 128.706, 128.469, 128.351, 77.936, 68.146, 61.881, 61.797, 54.895, 54.636, 24.304, 24.071, 24.029, 21.923, 20.016, 16.422;
³¹P NMR (121 MHz,CDCl₃) 29.853;

| | | | | | |
|---|---|---|---|---|---|
| C₁₇H₂₄NO₇P (385.36): | Ber. | C, 52.99; | H, 6.28; | N, 3.63; | P, 8.04 |
| | Gef. | C, 52.90; | H, 6.40; | N, 3.70; | P, 7.70. |

### Beispiel 14

### L-Homoalanin-4-yl(methyl)phosphinsäurehydrochlorid (L-PTC-Hydrochlorid)

0.50 g (1.4 mmol) (4S)-3-Benzyloxycarbonyl-4-{2-[ethoxy(methyl)phosphinyl]-ethyl}-1.3-oxazolidin-5-on (aus Beispiel 12) werden, wie in DE-A-3817956 beschrieben, durch Erhitzen in 6N Salzsäure verseift. Man erhält 0.25 g (82.1% der Theorie) L-Homoalanin-4-yl-(methyl)phosphinsäure-hydrochlorid als weißen Feststoff mit einem Schmelzpunkt von Fp 202 °C (Zers.).

Der Enantiomerenüberschuß, der mit Hilfe einer HPLC-Methode (J. Chromatogr. **1986**, *368*, 413) bestimmt wurde , beträgt 94,6 % ee.

### Beispiel 15

### L-2-Amino-4-phosphonobuttersäurehydrochlorid

1.00 g (2.6 mmol) (4S)-3-Benzyloxycarbonyl-4-[2-(diethoxyphosphinyl)-ethyl]-1.3-oxazolidin-5-on (aus Beispiel 13) werden, wie in DE-A-3817956 beschrieben, durch Erhitzen in 6N Salzsäure verseift. Man erhält 0.57 g (100% der Theorie) L-2-Amino-4-phosphonobuttersäure-hydrochlorid als hellbraunen Feststoff. Analytische Daten:
[α]_{D}²³= 17.3 ° (c= 0.548, 1N HCl);
- ¹H NMR (100 MHz,D₂O)δ=: 4.12 (t, 1, J= 5.5 Hz, C**H**(NH₂)COOH), 2.37-1.55 (m, 4, PC**H****₂**C**H****₂**CH)

## Patentansprüche

1. Optisch aktive, cyclische (2S)- und (2R)-ω-Halogen-2-aminoalkancarbonsäure-derivate der Formel I, 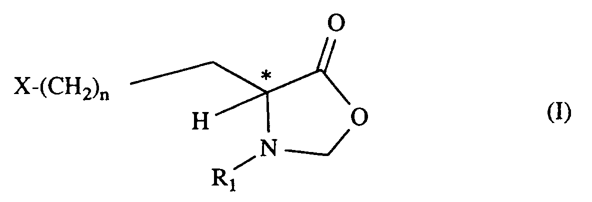 worin
R₁ (C₁-C₆)Alkyl, das unsubstituiert oder durch ein oder mehrere Reste aus der Gruppe Hydroxy, (C₁-C₄)Alkoxy, [(C₁-C₄)Alkyl]-carbonyl und [(C₁-C₄)Alkoxy]-carbonyl substituiert ist, (C₂-C₆)Alkenyl, (C₂-C₆)Alkinyl oder eine Gruppe der Formel R₂-CO- oder R₃-SO₂-,
R₂ (C₁-C₁₁)Alkyl, (C₆-C₁₃)Aryl oder substituiertes (C₆-C₁₃)Aryl, (C₁-C₆)Alkoxy, das unsubstituiert oder durch ein oder mehrere Reste, vorzugsweise einen Rest aus der Gruppe (C₆-C₁₃)Aryl und substituiertes (C₆-C₁₃)Aryl substituiert ist,
R₃ (C₁-C₄)Alkyl, Phenyl oder substituiertes Phenyl,
X Halogen und
n null oder eins
bedeuten,
ausgenommen die Verbindung der Formel I, worin R₁ = Benzyloxycarbonyl, X = Br und n = 1 ist.

2. Verbindungen der Formel I nach Anspruch 1, dadurch gekennzeichnet, daß
R₁ 1-Hydroxy-(C₁-C₆)-Alkyl, Methansulfonyl, Benzolsulfonyl, o-, m- oder p-Toluolsulfonyl oder eine Gruppe der Formel R₂-CO-,
R₂ (C₁-C₁₁)Alkyl, (C₆-C₁₂)Aryl oder substituiertes (C₆-C₁₂)Aryl, (C₁-C₄)Alkoxy, das unsubstituiert oder durch ein oder mehrere Reste aus der Gruppe Phenyl und substituiertes Phenyl substituiert ist,
X Chlor, Brom oder Iod und
n null oder eins bedeuten.

3. Verbindungen nach Anspruch 1 oder 2, dadurch gekennzeichnet daß
R₁ 1-Hydroxy-(C₁-C₄)-Alkyl, Methansulfonyl, Benzolsulfonyl, p-Toluolsulfonyl, Methoxycarbonyl, Ethoxycarbonyl, tert.-Butoxycarbonyl oder Benzyloxycarbonyl bedeutet.

4. Verbindungen nach einem der Ansprüche 1 bis 3, dadurch gekennzeichnet daß sie optisch aktive (2S)-Verbindungen der Formel I_{S} 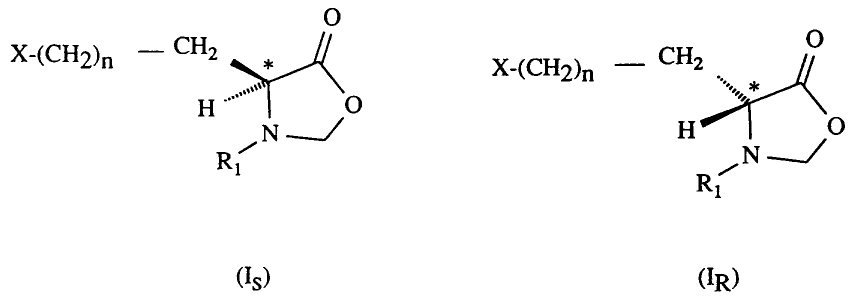 worin R₁, X und n wie in Formel I nach Ansprüchen 1, 2 bzw. 3 definiert sind, oder Gemische der Isomeren I_{S} und I_{R} mit einem Gehalt von mehr als 80% an der Verbindung der Formel I_{S} sind.

5. Verfahren zur enantioselektiven Synthese neuer und bekannter, optisch aktiver ω-Halogen-2-amino-alkancarbonsäure-derivaten der Formel I, 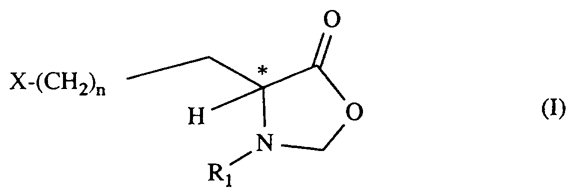 worin
R₁ (C₁-C₆)Alkyl, das unsubstituiert oder durch ein oder mehrere Reste aus der Gruppe Hydroxy, (C₁-C₄)Alkoxy, [(C₁-C₄)Alkyl]-carbonyl und [(C₁-C₄)Alkoxy]-carbonyl substituiert ist, (C₂-C₆)Alkenyl, (C₂-C₆)Alkinyl oder eine Gruppe der Formel R₂-CO- oder R₃-SO₂-,
R₂ (C₁-C₁₁)Alkyl, (C₆-C₁₃)Aryl oder substituiertes (C₆-C₁₃)Aryl, (C₁-C₆)Alkoxy, das unsubstituiert oder durch ein oder mehrere Reste aus der Gruppe (C₆-C₁₃)Aryl und substituiertes (C₆-C₁₃)Aryl substituiert ist,
R₃ (C₁-C₄)Alkyl, Phenyl oder substituiertes Phenyl,
X Halogen und
n null oder eins bedeuten,
dadurch gekennzeichnet, daß man
a) im Falle der Verbindungen der Formel I_{S} ein optisch aktives Diacylperoxid der allgemeinen Formel II_{S}, worin R₁ und n die gleiche Bedeutung wie in Formel I besitzen, in einem nitrilgruppenhaltigen Lösungsmittel in Gegenwart eines kupferhaltigen Katalysators mit einem Halogenierungsmittel DX umsetzt und die Verbindung der Formel I_{S} aus dem entstehenden Gemisch der Verbindungen I_{S} und III_{S} isoliert 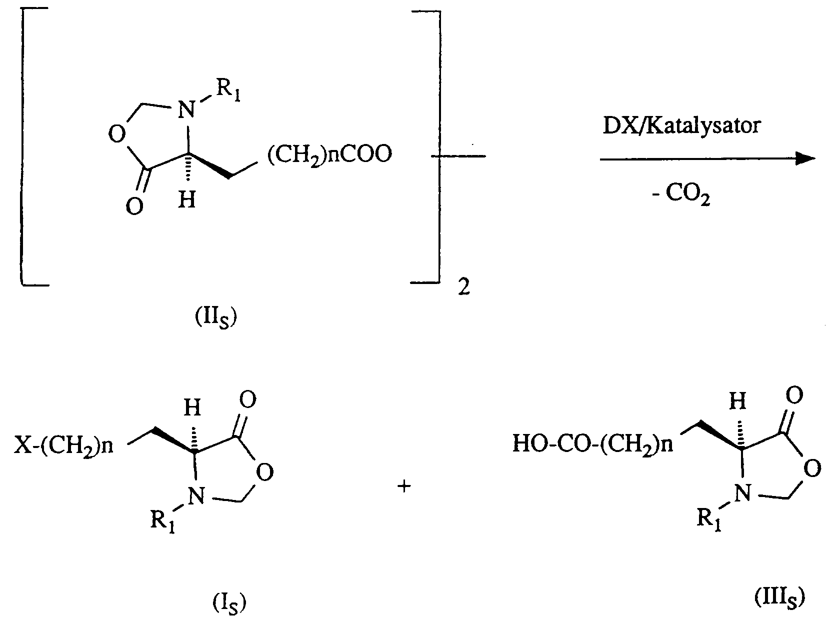 oder
b) im Falle der Verbindungen der genannten Formel I_{R}, oder Gemischen der Verbindungen der Formeln I_{R} und I_{S} ein optisch aktives Diacylperoxid II_{R}, das der Verbindung der unter a) genannten Formel II_{S} entspricht, jedoch anstelle der (S)-Konfiguration am jeweiligen asymmetrischen C-Atom die (R)-Konfiguration hat, bzw. ein entsprechendes Gemisch der Diacylperoxide II_{R} und II_{S} einsetzt und wie unter a) umsetzt und die Verbindung der Formel I_{R} bzw. das Gemisch der Verbindungen der Formel I_{R} und I_{S}isoliert.

6. Verfahren nach Anspruch 5, dadurch gekennzeichnet, daß
R₁ 1-Hydroxy-(C₁-C₆)-Alkyl, Methansulfonyl, Benzolsulfonyl, o-, m- oder p-Toluolsulfonyl oder eine Gruppe der Formel R₂-CO-,
R₂ (C₁-C₁₁)Alkyl, (C₆-C₁₂)Aryl oder substituiertes (C₆-C₁₂)Aryl, (C₁-C₄)Alkoxy, das unsubstituiert oder durch ein oder mehrere Reste aus der Gruppe Phenyl und substituiertes Phenyl substituiert ist,
X Chlor, Brom oder Iod und
n null oder eins bedeuten.

7. Verfahren nach Anspruch 5 oder 6, dadurch gekennzeichnet daß
R₁ 1-Hydroxy-(C₁-C₄)-Alkyl, Methansulfonyl, Benzolsulfonyl, p-Toluolsulfonyl, Methoxycarbonyl, Ethoxycarbonyl, tert.-Butoxycarbonyl oder Benzyloxycarbonyl bedeutet.

8. Verfahren nach einem der Ansprüche 5 bis 7, dadurch gekennzeichnet daß die Umsetzung der Acylperoxide der Formel II_{S} bzw. II_{R} in einem Temperaturbereich von -30 bis 100 °C und unterhalb der Zersetzungstemperatur des jeweiligen Peroxids vorgenommen wird.

9. Verfahren nach einem der Ansprüche 5 bis 8, dadurch gekennzeichnet daß die Umsetzung der Acylperoxide in Gegenwart eines Lösungsmittels aus der Gruppe enthaltend organische aliphatische Mono- und Dinitrile und aromatische Nitrile durchgeführt wird.

10. Verfahren nach einem der Ansprüche 5 bis 9, dadurch gekennzeichnet daß als Halogenierungsmittel DX elementares Halogen, Phosphor-V-halogenide oder Kupferhalogenide und als Katalysatoren Kupferverbindungen Kupfer-I-oxid oder Kupferhalogenide eingesetzt werden.

11. Diacylperoxide der allgemeinen Formel II, 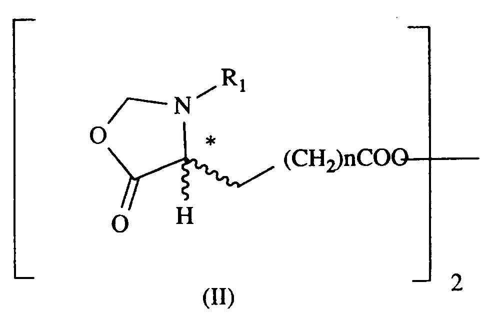 worin
R₁ und n wie in Formel I nach Anspruch 1 definiert sind.

12. Verfahren zur Herstellung der Verbindungen der Formel II nach Anspruch 11, dadurch gekennzeichnet, daß die den Diacylperoxiden entsprechenden Acylchloride mit Wasserstoffperoxid umgesetzt werden.

13. Verfahren zur Herstellung von Derivaten optisch aktiver, phosphorhaltiger α-Aminosäuren der allgemeinen Formel V, 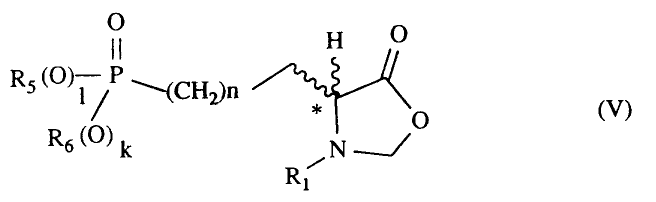 worin
R₅, R₆ unabhängig voneinander (C₁-C₈)-Alkyl, das unsubstituiert oder durch ein oder mehrere Reste aus der Gruppe Halogen substituiert ist, (C₃-C₈)-Cycloalkyl, (C₆-C₁₃)-Aryl oder substituiertes (C₆-C₁₃)-Aryl,
k null oder eins und
l null oder eins
bedeuten und R₁ und n die bei Formel I nach Anspruch I genannte Bedeutung haben, dadurch gekennzeichnet, daß man eine Verbindung der Formel I nach Anspruch I mit einer Verbindung der Formel IV, 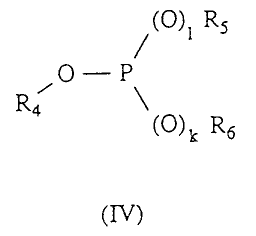 worin
R₄ Trialkylsilyl, (C₁-C₈)-Alkyl, das unsubstituiert oder durch ein oder mehrere Reste aus der Gruppe Halogen substituiert ist, (C₃-C₈)-Cycloalkyl, (C₆-C₁₃)-Aryl oder substituiertes (C₆-C₁₃)-Aryl
bedeutet und R₁, R₅, R₆, l, k und n wie bei Formel V definiert sind, analog den bekannten Bedingungen einer Arbusov-Reaktion umsetzt.

## Claims

1. An optically active, cyclic (2S)- or (2R)-ω-halo-2-aminoalkanecarboxylic acid derivative of the formula I, 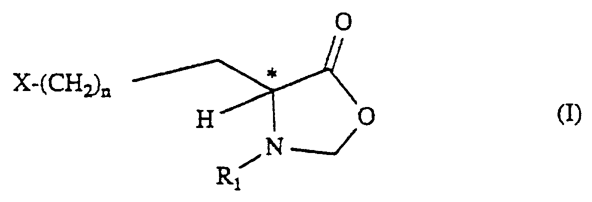 in which
R₁ is (C₁-C₆)alkyl which is unsubstituted or is substituted by one or more radicals selected from the group comprising hydroxyl, (C₁-C₄)alkoxy, [(C₁-C₄)alkyl]carbonyl and [(C₁-C₄)alkoxy]carbonyl; or is (C₂-C₆)alkenyl, (C₂-C₆)alkynyl or a group of the formula R₂-CO- or R₃-SO₂-,
R₂ is (C₁-C₁₁)alkyl, (C₆-C₁₃)aryl or substituted (C₆-C₁₃)aryl, (C₁-C₆)alkoxy, which is unsubstituted or substituted by one or more radicals, preferably a radical selected from the group comprising (C₆-C₁₃)aryl and substituted (C₆-C₁₃)aryl,
R₃ is (C₁-C₄)alkyl, phenyl or substituted phenyl,
X is halogen, and
n is zero or one,
except for the compound of the formula I in which R₁ = benzyloxycarbonyl, X = Br and n = 1.

2. A compound of the formula I as claimed in claim 1, wherein
R₁ is 1-hydroxy(C₁-C₆)alkyl, methanesulfonyl, benzenesulfonyl, o-, m- or p-toluenesulfonyl or a group of the formula R₂-CO-,
R₂ is (C₁-C₁₁)alkyl, (C₆-C₁₂)aryl or substituted (C₆-C₁₂)aryl, (C₁-C₄)alkoxy, which is unsubstituted or is substituted by one or more radicals selected from the group comprising phenyl and substituted phenyl,
X is chlorine, bromine or iodine and
n is zero or one.

3. A compound as claimed in claim 1 or 2, wherein
R₁ is 1-hydroxy(C₁-C₄)alkyl, methanesulfonyl, benzenesulfonyl, p-toluenesulfonyl, methoxycarbonyl, ethoxycarbonyl, tert-butoxycarbonyl or benzyloxycarbonyl.

4. A compound as claimed in one of claims 1 to 3, which is an optically active (2S) compound of the formula I_{S} 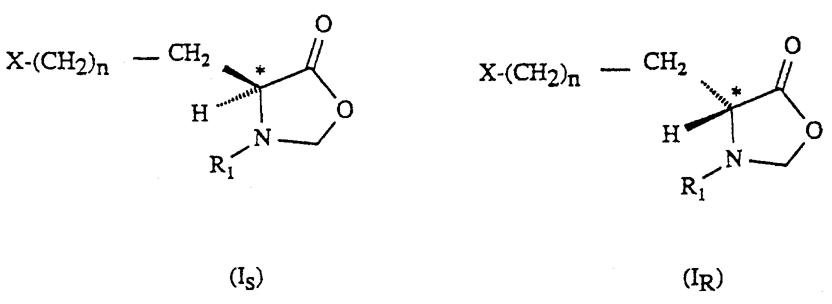 in which R₁, X and n are defined as in formula I as claimed in claim 1, 2 or 3,
or is a mixture of the isomers I_{S} and I_{R} having a content of more than 80% of the compound of the formula I_{S}.

5. A process for the enantioselective synthesis of novel and known optically active ω-halo-2-aminoalkanecarboxylic acid derivatives of the formula I, 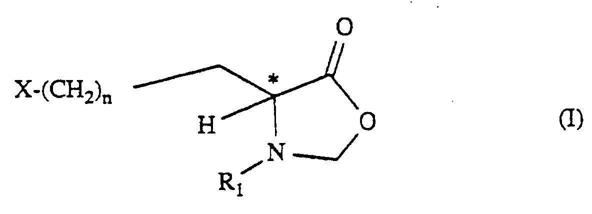 in which
R₁ is (C₁-C₆)alkyl which is unsubstituted or is substituted by one or more radicals selected from the group comprising hydroxyl, (C₁-C₄)alkoxy, [(C₁-C₄)alkyl]carbonyl and [(C₁-C₄)alkoxy]carbonyl; or is (C₂-C₆)alkenyl, (C₂-C₆)alkynyl or a group of the formula R₂-CO- or R₃-SO₂-,
R₂ is (C₁-C₁₁)alkyl, (C₆-C₁₃)aryl or substituted (C₆-C₁₃)aryl, (C₁-C₆)alkoxy, which is unsubstituted or substituted by one or more radicals selected from the group comprising (C₆-C₁₃)aryl and substituted (C₆-C₁₃)aryl,
R₃ is (C₁-C₄)alkyl, phenyl or substituted phenyl,
X is halogen, and
n is zero or one,
which comprises
a) in the case of the compound of the formula I_{S} mentioned, reacting an optically active diacyl peroxide of the formula II_{S} in which R₁ and n have the same meaning as in formula I, in a nitrile group-containing solvent in the presence of a copper-containing catalyst with a halogenation agent DX and isolating the compound of the formula I_{S} from the resulting mixture of the compounds I_{S} and III_{S} 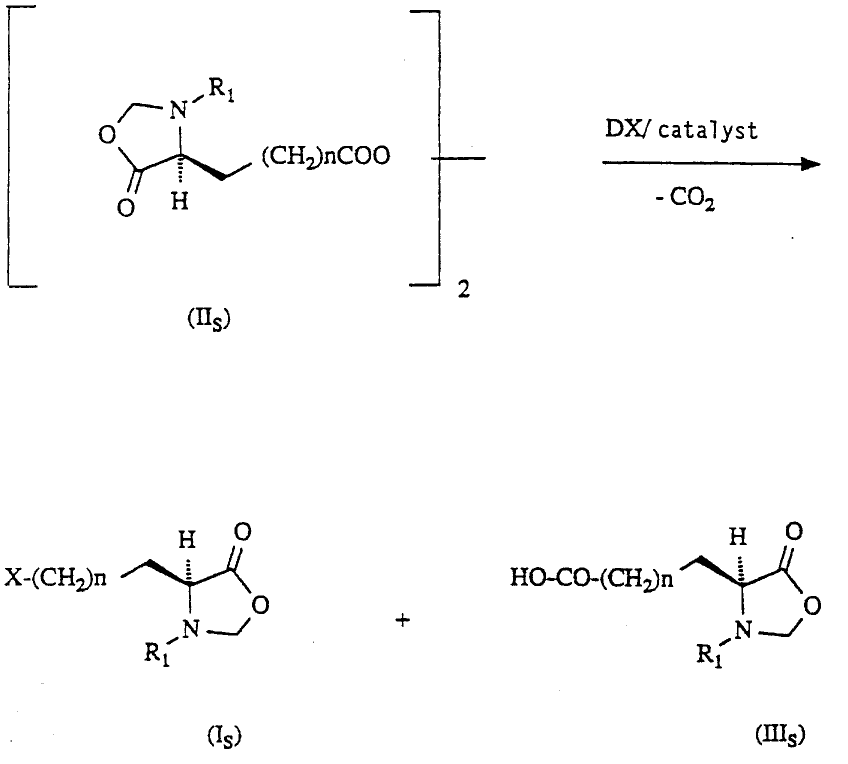 or
b) in the case of the compound of the formula I_{R} which corresponds to the formula I_{S} mentioned under a) but which has the (R) configuration at the asymmetric carbon atom instead of the (S) configuration, or a mixture of the compounds of the formulae I_{R} and/or I_{S}, using an optically active diacyl peroxide II_{R}, which corresponds to the compound of the formula II_{S} mentioned under a), but which instead of the (S) configuration at the respective asymmetric carbon atom has the (R) configuration, or a corresponding mixture of the diacyl peroxides II_{R} and II_{S}, and reacting them as under a) and isolating the compound of the formula I_{R} or the mixture of the compounds of the formulae I_{R} and I_{S}.

6. The process as claimed in claim 5, wherein
R₁ is 1-hydroxy(C₁-C₆)alkyl, methanesulfonyl, benzenesulfonyl, o-, m- or p-toluenesulfonyl or a group of the formula R₂-CO-,
R₂ is (C₁-C₁₁)alkyl, (C₆-C₁₂)aryl or substituted (C₆-C₁₂)aryl, (C₁-C₄)alkoxy, which is unsubstituted or is substituted by one or more radicals selected from the group comprising phenyl and substituted phenyl,
X is chlorine, bromine or iodine and
n is zero or one.

7. The process as claimed in claim 5 or 6, wherein
R₁ is 1-hydroxy(C₁-C₄)alkyl, methanesulfonyl, benzenesulfonyl, p-toluenesulfonyl, methoxycarbonyl, ethoxycarbonyl, tert-butoxycarbonyl or benzyloxycarbonyl.

8. The process as claimed in one of claims 5 to 7, wherein the reaction of the acyl peroxides of the formula II_{S} or II_{R} is carried out in a temperature range from -30 to 100°C and below the decomposition temperature of the respective peroxide.

9. The process as claimed in one of claims 5 to 8, wherein the reaction of the acyl peroxides is carried out in the presence of a solvent selected from the group comprising organic aliphatic mono- and dinitriles and aromatic nitriles.

10. The process as claimed in one of claims 5 to 9, wherein the halogenation agent DX used is elementary halogen, phosphorous(V) halides or copper halides and the catalysts used are copper compounds copper(I) oxide or copper halides.

11. A diacyl peroxide of the formula II, 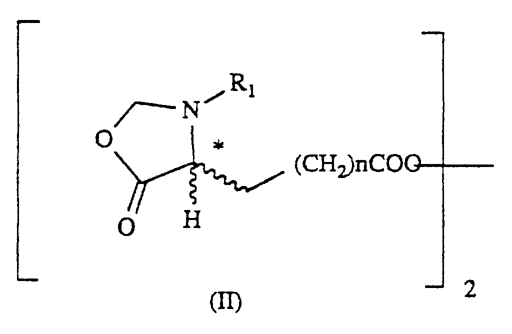 in which
R₁ and n are defined as in claim 1.

12. A process for the preparation of a compound of the formula II as claimed in claim 11, which comprises reacting the acyl chloride corresponding to the diacyl peroxide with hydrogen peroxide.

13. A process for the preparation of derivatives of an optically active phosphorus-containing α-amino acid of the formula V, 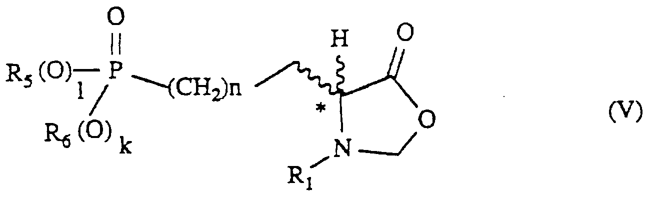 in which
R₅, R₆ are, independently of each other, (C₁-C₈)-alkyl which is unsubstituted or is substituted by one or more radicals from the halogen group, or is (C₃-C₈)-cycloalkyl, (C₆-C₁₃)aryl or substituted (C₆-C₁₃)aryl,
k is zero or one and
l is zero or one
and R₁ and n have the meanings given under formula I as claimed in claim 1, which comprises reacting a compound of the formula I as claimed in claim 1 with a compound of formula IV, 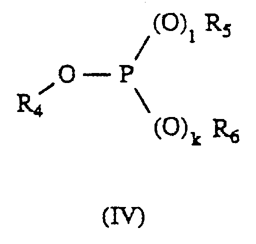 in which
R₄ is trialkylsilyl, (C₁-C₈)alkyl, which is unsubstituted or is substituted by one or more radicals from the halogen group, or is (C₃-C₈)cycloalkyl, (C₆-C₁₃)aryl or substituted (C₆-C₁₃)aryl
and R₁, R₅, R₆, l, k and n are defined as under formula V,
analogously to the known conditions of an Arbusov reaction.

## Revendications

1. Dérivés cycliques, optiquement actifs, d'acides (2S)- et (2R)-ω-halogéno-2-aminoalcanecarboxyliques de formule (I) 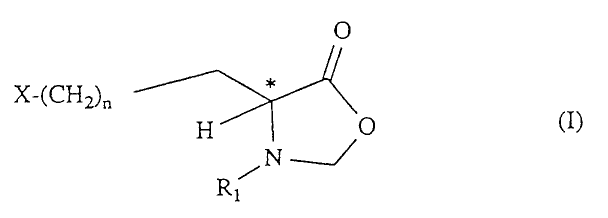 dans laquelle
R1 est un radical alkyle en C₁-C₆, qui est non substitué ou est substitué par un ou plusieurs substituants choisis parmi l'ensemble comprenant les groupes hydroxy, alcoxy en C₁-C₄, (alkyle en C₁-C₄)-carbonyle et (alcoxy en C₁-C₄)-carbonyle, alcényle en C₂-C₆, alcynyle en C₂-C₆, ou un groupe de formule R₂-CO- ou R₃SO₂-,
R₂ est un radical alkyle en C₁-C₁₁, aryle en C₆-C₁₃ ou aryle en C₆-C₁₃ substitué, alcoxy en C₁-C₆ qui est non substitué ou est substitué par un ou plusieurs substituants, de préférence un substituant, choisis parmi l'ensemble comprenant les groupes aryle en C₆-C₁₃ et aryle en C₆-C₁₃ substitués,
R₃ est un radical alkyle en C₁-C₄, phényle ou phényle substitué,
X est un halogène, et
n vaut zéro ou un,
à l'exception du composé de formule (I) dans laquelle R₁ est le radical benzyloxycarbonyle, X est Br et n vaut 1.

2. Composés de formule (I) selon la revendication 1, caractérisés en ce que
R₁ est un radical 1-hydroxyalkyle en C₁-C₆, méthanesulfonyle, benzènesulfonyle, o-, m- ou p-toluènesulfonyle ou un groupe de formule R₂-CO-, R₂ est un radical alkyle en C₁-C₁₁, aryle en C₆-C₁₂ ou
aryle en C₆-C₁₂ substitué, alcoxy en C₁-C₄ qui est non substitué ou est substitué par un ou plusieurs substituants choisis parmi l'ensemble comprenant les groupes phényle et phényle substitué,
X est le chlore, le brome ou l'iode, et
n vaut zéro ou un.

3. Composés selon la revendication 1 ou 2, caractérisés en ce que
R₁ est un radical 1-hydroxyalkyle en C₁-C₄, méthanesulfonyle, benzènesulfonyle, p-toluènesulfonyle, méthoxycarbonyle, éthoxycarbonyle, tert-butoxycarbonyle ou benzyloxycarbonyle.

4. Composés selon l'une des revendications 1 à 3, caractérisés en ce qu'il s'agit de composés (2S) optiquement actifs de formule IS 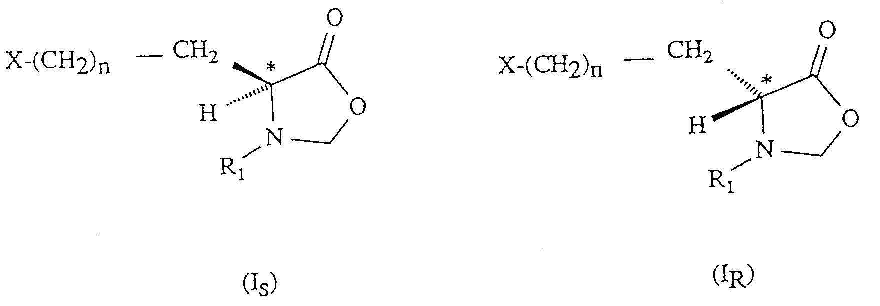 dans laquelle R₁, X et n ont les significations données dans la formule (I), respectivement dans les revendications 1, 2 et 3, ou encore de mélanges des isomères I_{S} et I_{R} contenant plus de 80 % du composé de formule I_{S}.

5. Procédé de synthèse énantiosélective de dérivés nouveaux et connus optiquement actifs d'acides ω-halogéno-2-aminoalcanecarboxyliques de formule I, 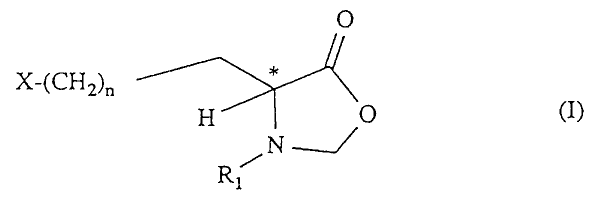 dans laquelle
R₁ est un radical alkyle en C₁-C₆, qui est non substitué ou est substitué par un ou plusieurs substituants choisis parmi l'ensemble comprenant les groupes hydroxy, alcoxy en C₁-C₄, (alkyle en C₁-C₄)-carbonyle et (alcoxy en C₁-C₄)-carbonyle, alcényle en C₂-C₆, alcynyle en C₂-C₆ ou un groupe de formule R₂-CO- ou R₃SO₂-,
R₂ est un radical alkyle en C₁-C₁₁, aryle en C₆-C₁₃ ou aryle en C₆-C₁₃ substitué, alcoxy en C₁-C₆ qui est non substitué ou est substitué par un ou plusieurs substituants choisis parmi l'ensemble comprenant les groupes aryle en C₆-C₁₃ et aryle en C₆-C₁₃ substitués,
R₃ est un radical alkyle en C₁-C₄, phényle ou phényle substitué,
X est un halogène, et
n vaut zéro ou un,
caractérisé en ce que
a) dans le cas des composés de formule I_{S}, on fait réagir un peroxyde de diacyle optiquement actif de formule générale II_{S}, dans laquelle R₁ et n ont les mêmes significations que dans la formule (I), dans un solvant contenant des groupes nitriles, en présence d'un catalyseur contenant du cuivre, avec un agent d'halogénation DX, et on isole le composé de formule I_{S} du mélange obtenu des composés I_{S} et III_{S} 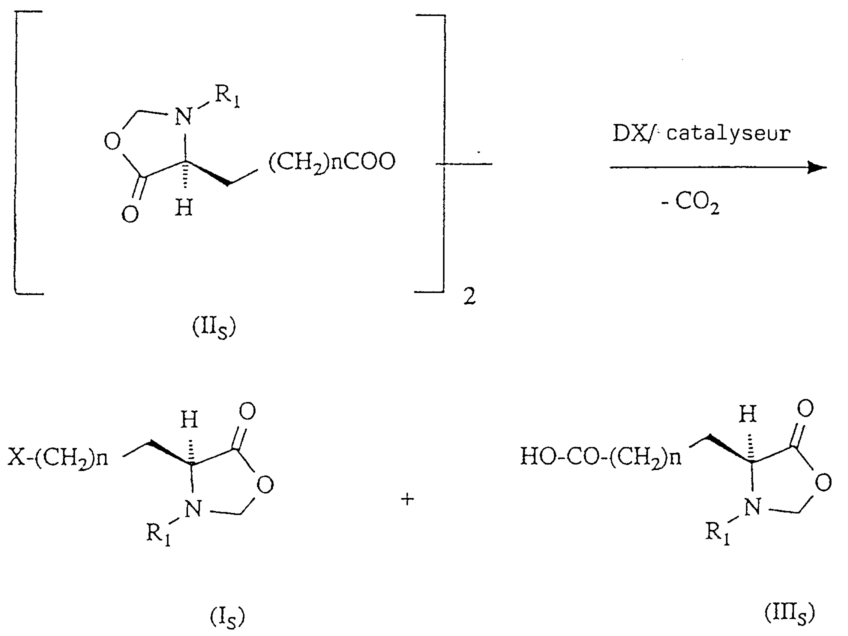 ou
b) Dans le cas des composés ayant la formule I_{R} mentionnée ci-dessus, ou de mélanges des composés de formules I_{R} et I_{S}, on utilise un peroxyde de diacyle optiquement actif II_{R}, qui correspond au composé ayant la formule II_{S} donnée en a), mais qui, au lieu de la configuration (S), a au niveau de l'atome de carbone asymétrique correspondant la configuration (R), ou encore un mélange correspondant des peroxydes de diacyle II_{R} et II_{S}, et on les fait réagir comme en a), et on isole le composé de formule IR ou le mélange des composés de formule I_{R} et I_{S}.

6. Procédé selon la revendication 5, caractérisé en ce que
R₁ est un radical 1-hydroxyalkyle en C₁-C₆, méthanesulfonyle, benzènesulfonyle, o-, m- ou p-toluènesulfonyle, ou un groupe de formule R₂-CO-,
R₂ est un radical alkyle en C₁-C₁₁, aryle en C₆-C₁₂ ou aryle en C₆-C₁₂ substitué, alcoxy en C₁-C₄ qui est non substitué ou est substitué par un ou plusieurs substituants choisis parmi l'ensemble comprenant les groupes phényle et phényle substitué,
X est le chlore, le brome ou l'iode, et
n vaut zéro ou un.

7. Procédé selon la revendication 5 ou 6, caractérisé en ce que
R₁ est un radical 1-hydroxyalkyle en C₁-C₄, méthanesulfonyle, benzènesulfonyle, p-toluènesulfonyle, méthoxycarbonyle, éthoxycarbonyle, tert-butoxycarbonyle ou benzyloxycarbonyle.

8. Procédé selon l'une des revendications 5 à 7, caractérisé en ce que la réaction des peroxydes d'acyle de formule II_{S} ou II_{R} est mise en oeuvre sur l'intervalle de températures de -30 à 100°C, et en-dessous de la température de décomposition du peroxyde considéré.

9. Procédé selon l'une des revendications 5 à 8, caractérisé en ce que la réaction des peroxydes d'acyle est mise en oeuvre en présence d'un solvant choisi parmi l'ensemble comprenant les mono- et dinitriles aliphatiques organiques et les nitriles aromatiques.

10. Procédé selon l'une des revendications 5 à 9, caractérisé en ce qu'on utilise comme agent d'halogénation DX un halogène élémentaire, des halogénures de phosphore-V ou des halogénures de cuivre et comme catalyseurs des composés du cuivre, de l'oxyde de cuivre-I ou des halogénures de cuivre.

11. Peroxydes de diacyle de formule générale (II) 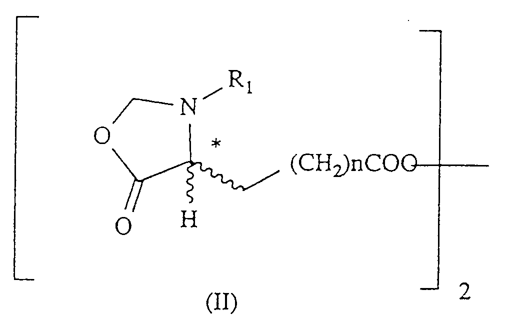 dans laquelle
R₁ et n ont les significations données dans la formule (I) selon la revendication 1.

12. Procédé pour préparer les composés de formule (II) selon la revendication 11, caractérisé en ce qu'on fait réagir les chlorures d'acyle correspondant aux peroxydes de diacyle avec du peroxyde d'hydrogène.

13. Procédé pour préparer des dérivés d'acides α-aminés phosphorés et optiquement actifs de formule générale (V) 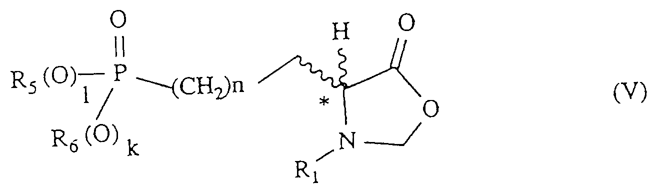 dans laquelle
R₅ et R₆, indépendamment l'un de l'autre, sont chacun un radical alkyle en C₁-C₈, qui est non substitué ou est substitué par un ou plusieurs substituants choisis parmi l'ensemble des halogènes, représente aussi cycloalkyle en C₃-C₈, aryle en C₆-C₁₃ ou aryle en C₆-C₁₃ substitué,
k vaut zéro ou un, et
l vaut zéro ou un, et
et R1 et n ont les significations données pour la formule (I) de la revendication 1, caractérisé en ce qu'on fait réagir, d'une manière analogue aux conditions connues d'une réaction d'Arbusov, un composé de formule (I) selon la revendication 1 avec un composé de formule (IV) 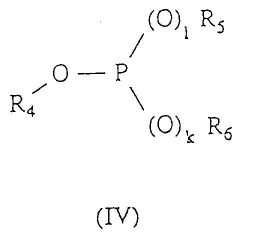 dans laquelle
R₄ est un radical trialkylsilyle, alkyle en C₁-C₈, qui est non substitué ou est substitué par un ou plusieurs radicaux choisis parmi les halogènes, cycloalkyle en C₃-C₈, aryle en C₆-C₁₃ ou aryle en C₆-C₁₃ substitué,
et R₁, R₅, R₆, l, k et n ont les significations données pour la formule (V).
